Europäisches Patentamt

European Patent Office

Office européen des brevets

(19)

(11) Publication number : **0 337 895 B1**

(12) # EUROPEAN PATENT SPECIFICATION

(45) Date of publication of patent specification :
07.01.93 Bulletin 93/01

(51) Int. Cl.⁵ : **A61M 5/14,** F16K 7/10, G05D 7/01

(21) Application number : **89401044.6**

(22) Date of filing : **14.04.89**

(54) **Flow control device.**

(30) Priority : **15.04.88 JP 92854/88**
**23.05.88 JP 126846/88**

(43) Date of publication of application :
**18.10.89 Bulletin 89/42**

(45) Publication of the grant of the patent :
**07.01.93 Bulletin 93/01**

(84) Designated Contracting States :
**BE DE ES FR GB IT NL SE**

(56) References cited :
**CH-A- 662 635**
**DE-A- 2 653 415**
**US-A- 3 893 468**
**US-A- 4 406 440**

(73) Proprietor : **TERUMO KABUSHIKI KAISHA**
**No. 44-1, Hatagaya 2-chome, Shibuya-ku**
**Tokyo 151 (JP)**

(72) Inventor : **Nakada, Tsuneo**
**Terumo K.K. 1727-1, Tsuijiarai Showa-cho**
**Nakakoma-gun Yamanashi-Ken (JP)**

(74) Representative : **Joly, Jean-Jacques et al**
**CABINET BEAU DE LOMENIE 55, rue**
**d'Amsterdam**
**F-75008 Paris (FR)**

## Description

This invention relates to a flow control device used for controlling the rate of flow of liquid passing through a liquid supply tube of a liquid transfer set, such as a solution administration set.

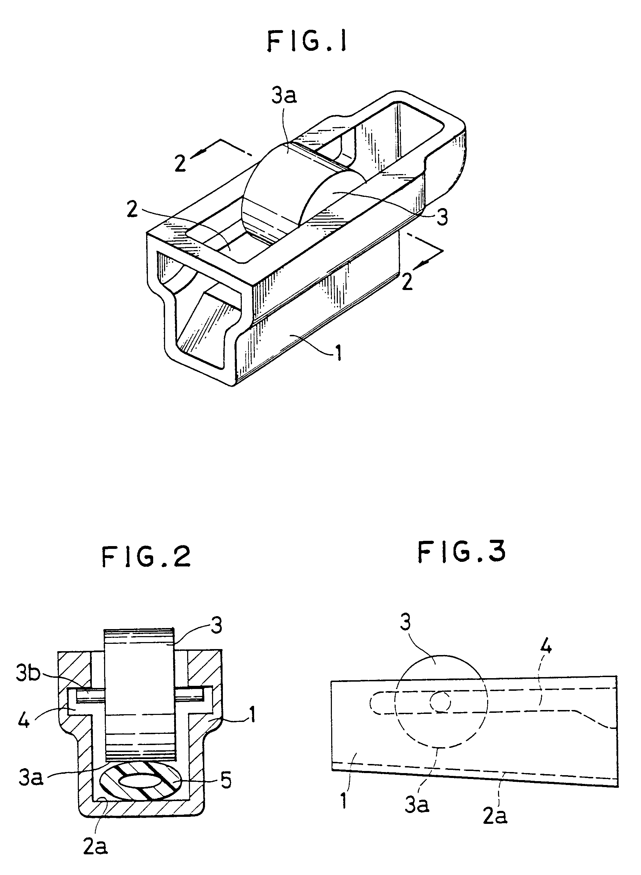

Figs. 1 to 3 show a prior art liquid flow device of this type. As is shown, the device comprises a housing 1 with a liquid supply tube insertion channel 2. Opposite side walls of the liquid supply tube insertion channel 2 are formed with bearing grooves 4. A manually movable roller 3 has its shaft 3b mounted in the bearing grooves 4 such that it is movable along the liquid supply tube insertion channel 2. The liquid supply tube insertion channel 2 has a bottom surface 2a which is inclined by a predetermined angle with respect to the bearing grooves 4. A liquid supply tube 5 is passed through the bottom surface 2a and periphery 3a of the roller 3. The roller 3 is manually moved to be closer to or remoter from the bottom surface 2a of the liquid supply tube insertion channel 2 to vary the extent of squeezing of the liquid supply tube 5 so as to control the rate of flow of liquid.

In the above prior art flow control device, the liquid supply tube 5 is made of a soft thermoplastic polymer such as polyvinyl chloride and is therefore subject to a phenomenon of creep. Squeezed opposite edges of the liquid supply tube initially have strong restoring force and tend to restore the initial state. In long use, however, the restoring force becomes weaker, so that the initially formed inner flow passage of the liquid supply tube 5 is changed in long use. This means that it is difficult to obtain a stable rate of flow.

In different aspect, with the prior art flow control device it is necessary to provide a wide range of rate of flow, from zero rate to a very high rate. Also, a certain limitation is imposed on the length of the housing 1 by considerations of the durability and operability. Therefore, it is difficult to reduce the inclination of the bottom surface 2a of the liquid supply tube insertion channel 2. This means that it is difficult to obtain flow control, particularly fine flow control.

Other prior art pertaining to the flow control device according to the invention includes Japanese Patent Disclosures 47-1540, 50-41374, 52-81718, 52108691, 52-142331, 53-103685, 53-144191, 53-148193, 54-2515, 54-10426, 54-10427, 54-60792, 54-108492, 55-72986, 58-30593, 61-41462, 50-125591, 50-125592 and 51-102392, Japanese Utility Model Disclosures 53-18089, 53-42499, 54-3096, 54-66697, 54-72398, 54183196, 55-2338, 57-12868, 57-188976, 57-194958, 57194959, 58-58150, 58-58151, 59-5939, 59-88536, 5992265, 61-151742, 61-156935, 61-180042, 61-180043 and 54-131395, Japanese Patent Publications 48-36526, 55-27986, 55-27987, 55-31351, 57-8342, 60-27870, 558573, 56-43578, 56-49013, 58-22224 and 58-44384, Japanese Utility Model Publications 51-45911 and 59-9642 and Japanese Patent Announcement 56-501619.

US-A-3 893 468 teaches the provision of ridges along the tube insertion channel to pinch the edges of the tube to improve grip. Finally, CH-A-662 635 discloses a flow control device that corresponds substantially to the preamble of claim 1.

## SUMMARY OF THE INVENTION

An object of the invention is to provide a flow control device, which provides a wide range of flow rates from zero rate to a very high rate with an ordinary housing length and permits accurate flow control, particularly in a fine flow control range.

To attain the above object of the invention, there is provided a flow control device as defined in claim 1.

With the flow control device having the above construction according to the invention, after mounting the liquid supply tube by inserting it in the liquid supply tube insertion channel, the rate of flow in the tube is controlled by moving the roller from one end toward the other end.

In its travel from one end to the other, the roller is made to cover successively or coarse adjustment range, a fine adjustment range, and a coarse adjustment range, with the length of the inclined surface portion corresponding to the fine adjustment range being greater than both of the lengths corresponding to the coarse adjustment range.

## BRIEF DESCRIPTION OF THE DRAWINGS

Fig. 1 is a perspective view showing a prior art flow control device;
Fig. 2 is a sectional view taken along line 2-2 in Fig. 1;
Fig. 3 is a side view showing the device shown in Fig. 1;
Fig. 4 is a perspective view showing a flow control device fitted with ridges along the opposite edges of the bottom of the liquid supply tube insertion channel;
Fig. 5 is a sectional view taken along line 5-5, to an enlarged scale, showing the device shown in Fig. 4 with a liquid supply tube mounted therein;

Fig. 6 is a fragmentary sectional view showing another flow control device in which ridges are fitted on the periphery of the roller wheel;

Fig. 7 is a perspective view showing an embodiment of the flow control device according to the invention;

Fig. 8 is a side view showing the device shown in Fig. 7, and

Fig. 9 is an schematic diagram on an exgaggerated scale showing the disposition of the of the first, second and third inclined surfaces of the embodiment shown in Fig. 8

## DETAILED DESCRIPTION OF THE PREFERRED EMBODIMENTS

Now, some preferred embodiments of the invention will be described with reference to the drawings.

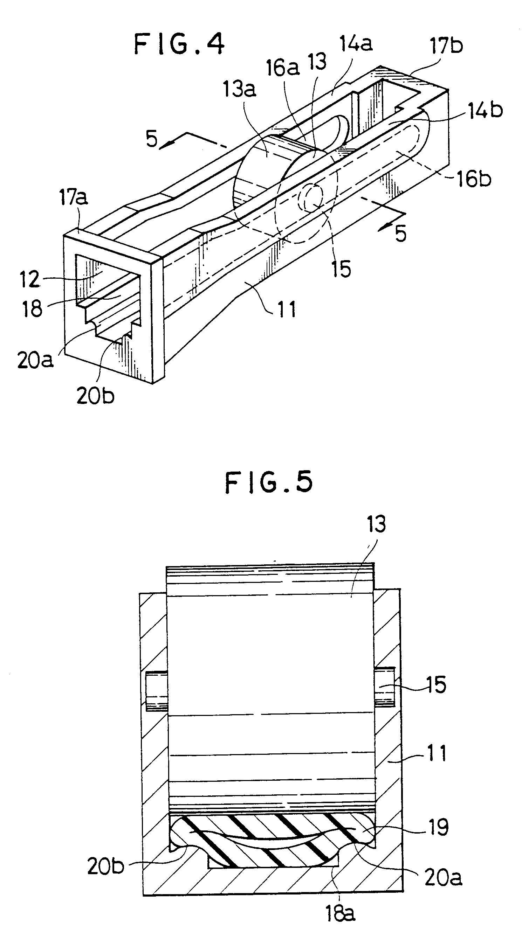

Fig. 4 is a perspective view showing flow control device fitted with ridges along opposite edges of the bottom of the liquid supply tube insertion channel. In the Figure, reference numeral 11 designates a substantially rectangular housing made of a hard polymer material. In the housing 11, groove-like space 12 is formed extending in its longitudinal direction. A manually movable roller 13 is mounted in the space 12 for movement in the longitudinal direction of the insertion channel. More specifically, opposed wall surfaces 14a and 14b of the housing 11 are formed with bearing grooves 16a and 16b to support and guide a shaft 15 of the roller 13 in the longitudinal direction of the housing 11. The roller 13 partly projects from the housing 11, and it can be rotated by operating its projecting portion with a finger. The longitudinally opposite ends of the housing 11 are provided at the top with reinforcement frames 17a and 17b each intervening between the side walls 14a and 14b. When the roller 13 is moved to one end of the space 12, the shaft 15 strikes ends of the bearing grooves 16a and 16b so that the roller can no further be moved.

It will be seen that the roller 13 can be moved in the space 12 along the bearing grooves 16a and 16b in the longitudinal direction of the housing 11. The liquid supply tube insertion channel 18 is defined such that it constitutes the bottom of the space 12, and it extends in the longitudinal direction thereof and faces the periphery 13a of the roller 13. The bottom surface of the liquid supply tube insertion channel 18 consitutes an inclined surface inclined at a fixed angle to the bearing grooves 16a and 16b from one end to the other end of the bearing grooves. Thus, as the roller 13 is manually moved from one end toward the other end of the bearing grooves 16a and 16b, its periphery becomes closer and closer to the bottom of the liquid supply tube insertion channel 18 to squeeze the liquid supply tube inserted in the insertion channel 18, thus effecting the flow rate control.

Fig. 5 is a sectional view taken along line 5-5 in Fig. 4, showing the device with a liquid supply tube 19 made of a soft polymer material mounted in the liquid supply tube insertion channel 18. Opposite edges of the bottom 18a of the liquid supply tube insertion channel 18 are provided with ridges 20a and 20b serving as liquid supply tube urging means, which extend in the longitudinal direction and have a concave top surface. The liquid supply tube 19 is more strongly squeezed at its opposite end portions in sectional view than its central portion between the ridges 20a and 20b on one hand and the periphery 13a of the roller 13 on the other hand. In this way, the liquid supply tube 19 is reliably secured, and changes in the rate of flow in the liquid supply tube 19 in long use are prevented.

In specific numerical figures, when using a roller with an outer diameter of 14 mm and a width of 4.7 mm as the roller 13 and a liquid supply tube made of polyvinyl chloride and with an outer diameter 3.3 mm and a wall thickness of 0.6 mm as the liquid supply tube 19, it is desired to set the width x of the roller 13 and width y of the liquid supply tube insertion channel 18 of the housing 11 to be in a relation:

$$0.2 \text{ mm} < x - y < 0.4 \text{ mm}$$

In other words, strong contact between each side surface of the roller 13 and corresponding side wall of the liquid supply tube insertion channel 18 precludes looseness of the roller 13 due to the elasticity of the liquid supply tube 19 and prevents changes in the size and shape of the liquid supply tube insertion channel 18 in long use.

It is also possible to prevent changes in the size and shape of the liquid supply tube insertion channel 18 by setting the axial length $x'$ of the roller 13 and mounting width $y'$ of the roller shaft 15 in the housing 11 to be in a relation:

$$0.2 \text{ mm} < x' - y' < 0.4 \text{ mm}$$

The ridges 20a and 20b suitably have their height set to 0.2 to 0.8 mm, width to 0.5 to 1.0 mm and radius R of curvature to 0.3 to 1.0 mm.

With the flow control device having the above construction, after positioning the roller 13 at one end of the liquid supply tube insertion channel 18 the liquid supply tube 19 is inserted in the insertion channel 18 and set at a desired position. Then, the roller 13 is rotated with a finger. The roller 13 thus rolls over the liquid supply tube 19 toward the other end of the insertion channel 18. This movement of the roller 13 squeezes the liquid supply tube 19 and effect control of the rate of flow of liquid in the tube.

Since the opposite edges of the bottom of the liquid supply tube insertion channel 18 are provided with the ridges 20a and 20b, the liquid supply tube 19 is squeezed at its opposite end portions in sectional view where the phenomenon of creep is pronounced. Thus, the opposite end portions of the liquid supply tube noted above are reliably secured, and the phenomenon of creep is prevented.

Further, since the roller 13 is firmly engaged in the liquid supply tube insertion channel 18, the insertion channel 18 will never be changed in size or shape in long use. Thus, the liquid supply tube 19 is never deformed, and a stable rate of flow can be obtained.

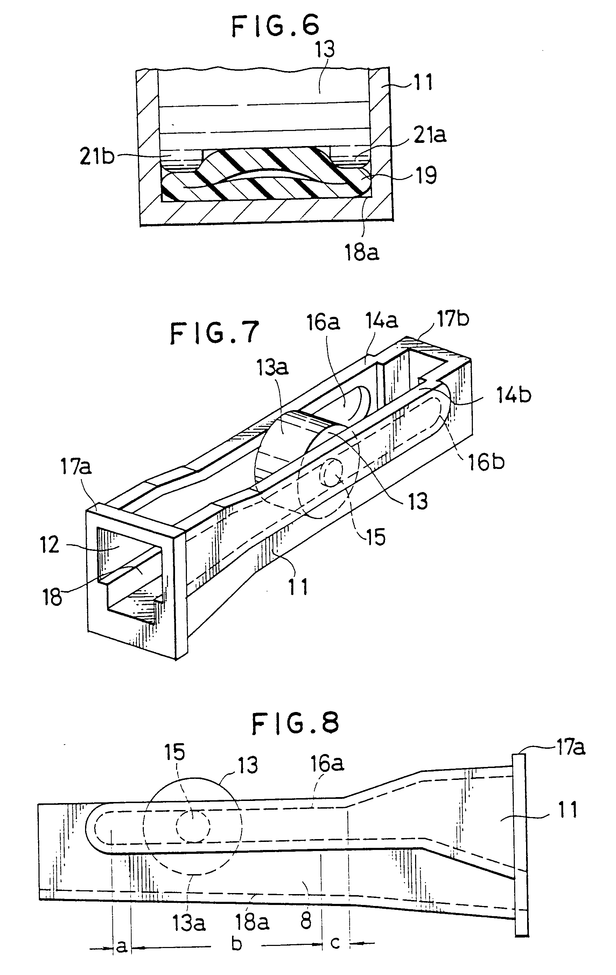

In the foregoing, the ridges 20a and 20b for prevention of creep are provided on the side of the liquid supply tube insertion channel 18. However, it is possible to provide the ridges on the side of the roller 13 as well, as shown in Fig. 6. More specifically, in this case ridges 21a and 21b are formed on opposite edge portions of the periphery 13a of the roller 13.

With this construction, the liquid supply tube 19 is squeezed between the ridges 21a and 21b on the roller 13 and opposite edges of the bottom surface 18a of the liquid supply tube insertion channel 18. In this way, creep of the liquid supply tube 19 can be prevented.

It is of course possible to provide ridges on opposite edges of the periphery 13a of the roller 13 and bottom surface 18a of the liquid supply tube insertion channel 18. These ridges may be formed separately from the housing and roller 13. These ridges, as urging means, may not be continuous and may have any desired shape so long as they can substantially prevent creep of opposite edges of the squeezed liquid supply tube 19.

A flow control device as shown in Fig. 4 was used, which comprised a manually movable roller made of ABS (acrylonitrile-buthadiene-styrene copolymer) and a housing made of polyethylene. A liquid supply tube made of polyvinyl chloride was mounted in the device for measuring the rate of change in number of drops. Opposite edges of the bottom surface of the liquid supply tube insertion channel of the housing were provided with ridges with a height of 0.5 mm, a width of 0.7 mm and a radius R of curvature of 0.6 mm.

Further, the width of the surface of the roller in contact with the housing was set to 4.7 mm, and the width of the liquid supply tube insertion channel on the housing side to 4.4 mm.

For the measurement of the rate of change in number of drops, a solution administration set as the liquid transfer set using the above flow control device and liquid supply tube was coupled to a dripping bottle containing a medicine. The liquid transfer rate was adjusted to $60 \pm 5$ drops per minute, and then the number of drops per minute was measured for 60 minutes. The maximum rate of change in number of drops was obtained as

$$\text{Maximum rate of change in number of drops } (\%)$$

$$= \frac{\left(\begin{array}{c}\text{Maximum or minimum} \\ \text{drop number in 60 min.}\end{array}\right) - \left(\begin{array}{c}\text{Initially set} \\ \text{drop number}\end{array}\right)}{\text{Initially set drop number}} \times 100$$

One drop was set to about 1/15 cc and to about 1/60 cc. Table 1 shows the results of measurement in case of about 1/15-cc drops, and Table 2 shows the results in case of about 1/60-cc drops:

Table 1

| | Average (%) | Maximum (%) | Minimum (%) |
|---|---|---|---|
| device without ridges (n=5) | -37.6 | -33.1 | -45.0 |
| device with ridges (n=30) | -5.3 | +4.3 | -11.7 |

Table 2

| | Average (%) | Maximum (%) | Minimum(%) |
|---|---|---|---|
| device without ridges (n=5) | -64.7 | -58.5 | -72.2 |
| device with ridges (n=30) | -6.6 | +22.2 | -37.0 |

It will be seen from the above results that with devices fitted with ridges, changes in number of drops are small compared to devices without ridges, that is, it is possible to obtain steady drop number accuracy at all time.

It is to be appreciated that when substantially continuous ridges are provided on opposite edges of the bottom surface of the liquid supply tube insertion channel in the housing and opposite edges of the periphery of the roller, it is possible to prevent flow rate changes that might otherwise occur due to creep of the liquid supply tube, and stable rate of flow can be obtained at all time.

There will now be described a control device according to the present invention, with reference to Figures 7, 8 and 9. Parts like those in Fig. 4 and 5 are designated by like reference numerals with omission of the description.

In the embodiment, the inclined surface of the bottom 18a of the liquid supply tube insertion channel 18 defines a portion for co-operating with the roller 13 in squeezing the liquid supply tube 19 to control the rate of flow of liquid in the tube, and, consists of three different inclined surfaces: (b) a first inclined surface, (c) a second inclined surface, and (a) a third inclined surface, continuous to one another and corresponding to respective rates of supply of liquid, as shown in Fig. 8. These inclined surfaces (a) to (c) have respective lengths and inclination angles as shown in Table 3.

Although the inclined surfaces (a) to (c) are shown to be linear in Fig. 8, actually they are curved in the borderline between adjacent ones of them.

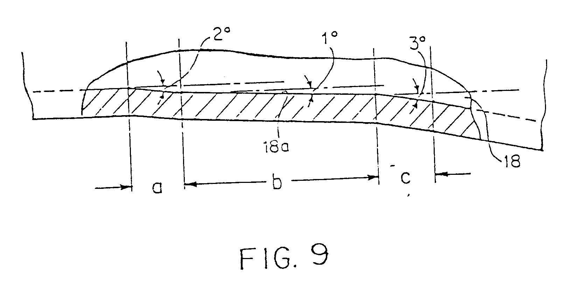

This fact is shown more clearly in Figure 9, which is a schematic diagram on an exaggerated scale illustrating the disposition of the slopes of the first, second and third inclined surfaces.

Table 3

| Portion | Length | Inclination angle |
|---|---|---|
| a | 2 mm | 2° |
| b | 20 mm | 1° |
| c | 4 mm | 3° |

Preferably, opposite edges of the inclined surfaces (a) to (c) of the bottom 18a of the liquid supply tube insertion channel 18 are provided with ridges 20a and 20b extending in the longitudinal direction and having a curved top surface. The opposite edges of the liquid supply tube 19 are squeezed more strongly than the central portion between the ridges 20a and 20b on one hand and the periphery 13a of the roller 13 on the other hand.

Thus, it is possible to reliably secure the liquid supply tube 19, prevent flow large changes in the liquid supply tube 19 in long time and improve the accuracy of the flow rate control.

With the flow control device having the above construction, the roller 13 is moved to one end of the liquid supply tube insertion channel 18 to stop the liquid supply. At this position, the inclined surface (a) of the bottom 18a of the liquid supply tube insertion channel 18 facing the periphery 13a of the roller 13 is inclined at a fixed angle of 2°.

Therefore, the liquid supply can be reliably stopped even if there are fluctuations of the thickness of the wall of the liquid supply tube 19. To supply liquid at a very slight rate, the roller 13 is moved to a position corresponding to the inclined surface (b) with a smaller angle of 1° than that of the liquid supply stop zone of the bottom 18a of the liquid supply tube insertion channel 18. In this way, it is possible to supply liquid at a slight rate. To supply liquid at a very high rate, the roller 13 is moved to a position correponding to the inclination

5

surface (c) with a greater inclination angle of 3° than that of the slight flow rate zone.

In the above embodiment the inclined surfaces (a) and (c) are provided as second and third inclined surfaces with a greater inclination angle than that of the inclined surface (b) as first inclined surface such that they terminate in to the opposite ends of the inclined surface (b). However, this is by no means limitative; for instance, it is possible to provide only a single second inclined surface terminating in either end of the first inclined surface. Further, although the creep prevention ridges 20a and 20b are provided on the side of the liquid supply tube insertion channel 18, it is possible to provide ridges on the side of the roller 13 as well.

Using the above embodiment of the device measurement of the drop number control range was conducted by also using a contrast device, which had the same housing length but with a constant liquid supply tube insertion channel bottom inclination surface angle of 2°.

The embodiment and contrast flow control devices were each assembled in a liquid transfer set (with a liquid supply tube with an outer diameter of 3.3 mm and a wall thickness of 0.6 mm and a drop size of approximately 1/15 cc on one case and approximately 1/60 cc in another case) and connected to a liquid transfer bag. Positions of the roller correponding to 0, 60 and 120 drops per minute were measured with a head of 1 m to obtain distances covered by the roller corresponding to 0 to 60 drops per minute and 60 to 120 drops per minute. The results are shown in Tables 4 and 5.

Table 4

(Drop size of approximately 1/15 cc, n=10)

Distance covered by roller (mm)

| | 0-60 drops/min Ave. (min. to max.) | 60-120 drops/min Ave. (min. to max.) |
|---|---|---|
| Embodiment | 4.5(2.5  8.5) | 2.5(0.5  6.3) |
| Contrast | 2.9(2.4  3.8) | 1.2(0.3  1.7) |

Table 5

(Drop size of approximately 1/60 cc, n=10)

Distance covered by roller (mm)

| | 0-60 drops/min Ave. (min. to max.) | 60-120 drops/min Ave. (min. to max.) |
|---|---|---|
| Embodiment | 4.3(1.8  8.5) | 1.0(0.3  2.1) |
| Contrast | 1.8(1.5  2.6) | 0.4(0.2  0.6) |

As is seen from the results, with the embodiment of the flow control device greater distance can be covered by the roller with the same housing length and same drop number change. This means that it is possible according to the invention to readily obtain adjustment to a desired position.

With the contrast flow control device having a constant liquid supply tube insertion channel bottom inclined surface angle, all flow rate range can not be ensured unless the inclination angle is reduced and housing length is increased to obtain the same level of distance covered by the roller as in the case of the embodiment (i.e., obtain the same readiness of adjustment as in the case of the embodiment).

As has been shown, with the above embodiment of the flow control device a portion of the bottom surface of the liquid supply tube insertion channel in the housing that co-operates with the roller for squeezing the liquid supply tube to control the flow rate therein consists of a first inclined surface providing a slight flow rate and a second inclined surface with a greater inclination angle than the first inclined surface, the distance covered by the roller can be extended to facilitate the flow rate control by reducing the inclination angle of the first inclined surface, while the second inclined surface corresponding to a high flow rate range permits flow rate control without spoiling the readiness of flow control in the slight flow rate range. It is thus possible to ensure all flow rate range with a compact housing length.

It can also be noted that the fluid flow control device of the invention does not preclude the provision of the ridges on opposite edges of the bottom of the liquid supply tube insertion channel in the housing and also on opposite edges of the roller periphery in contact with the liquid supply tube, it is possible to prevent changes in the flow rate in long use that might otherwise result from the phenomenon of creep in the liquid supply tube, thus further improving the accuracy of the flow control.

**Claims**

1.  A flow control device with a flexible liquid supply tube mounted therein such that said flexible liquid supply tube can be squeezed by virtue of its flexibility to vary the size of a flow path in said liquid supply tube to thereby control the rate of flow of liquid through said liquid supply tube,

    said flow control device comprising :

    a housing (11) having a liquid supply tube insertion channel (18), through which said liquid supply tube (19) is inserted, said housing having opposite side walls (14a, 14b) which have bearing grooves (16a, 16b) therein which extend in the longitudinal direction of said liquid supply tube insertion channel, said liquid supply tube insertion channel having a bottom (18a); and

    a roller (13) having a shaft (15) supported in said bearing grooves such that said roller is movable along said bearing grooves and along said liquid supply tube insertion channel from one end to the other end, said liquid supply tube in said liquid supply tube insertion channel being squeezed by the periphery of said roller,

    said bottom of the liquid supply tube insertion channel including first and second inclined surfaces (b, c) which are inclined with respect to said bearing grooves and which are selectively co-operative with said roller to squeeze said liquid supply tube for control of the rate of flow of liquid through said liquid supply tube, characterized in that it further comprises a third inclined surface (a) that is also inclined with respect to said bearing grooves and selectively co-operative with said roller to squeeze said liquid supply tube for control of the rate of flow of liquid through said liquid supply tube,

    said first inclined surface (b) being inclined at a given angle and being adapted for fine adjustment of rate flow of liquid through said supply tube, said second inclined surface (c) terminating at one end portion thereof at one end portion of said first inclined surface portion, said third inclined surface portion being inclined in the same direction as said first inclined surface but by a greater angle than said first inclined surface and being adapted for a coarser adjustment of the rate of liquid flow through said liquid supply tube, said third inclined surface portion (a) terminating at one end thereof at another end portion of said first inclined surface (b), such that said first inclined surface portion for said fine adjustment is located between said second and third inclined surface portions for said coarser adjustment; and the length of said first inclined surface portion being greater than the lengths of said second and third inclined surface portions.

2.  The flow control device according to claim 1, wherein the inclination angles of said first (b) and second (c) inclined surface portions are substantially 1° and 3°, respectively.

3.  The flow control device according to claim 1, wherein the inclination angle of said third (c) inclined surface portion is substantially 2°.

4.  The flow control device according to claim 1, wherein it further comprises urging means (20a, 20b, 21a, 21b) for urging opposite edges of said liquid supply tube inserted in said insertion channel extending in the longitudinal direction of said liquid supply tube.

5.  The flow control device according to claim 4, wherein said urging means is constituted by a pair of substantially continuous ridges (20a, 20b) extending along the opposite edges of the bottom (18a) of said liquid supply tube insertion channel (18) in the direction of movement of said roller (13) and in a range of movement of said roller.

6.  The flow control device according to claim 4, wherein said urging means is constituted by a pair of substantially continuous ridges (21a, 21b) formed on the opposite edges of the periphery of said roller (13) so as to be in contact with said liquid supply tube (19).

7. The flow control device according to claim 4, wherein said urging means is constituted by a plurality of substantially continuous ridges (20a, 20b, 21a, 21b) formed on opposite edges of the bottom (18a) of said liquid supply tube insertion channel (18) extending in the direction of movement of said roller (13) and also on opposite edges of the periphery of said roller so as to be in contact with said liquid supply tube (19).

**Patentansprüche**

1. Strömungsregler mit einem darin eingesetzten flexiblen Flüssigkeitsspeiseschlauch, derart, daß der Flüssigkeitsspeiseschlauch aufgrund seiner Flexibilität zusammendrückbar oder verquetschbar ist, um die Größe eines Strömungswegs im Flüssigkeitsspeiseschlauch zu variieren und damit die Strömungsmenge von Flüssigkeit durch den Flüssigkeitsspeiseschlauch zu regeln,

wobei der Strömungsregler umfaßt:

ein Gehäuse (11) mit einem Flüssigkeitsspeiseschlauch-Einsetzprofil (18), durch welches der Flüssigkeitsspeiseschlauch (19) eingesetzt ist, wobei das Gehäuse gegenüberliegende Seitenwände (14a, 14b) mit darin vorgesehenen Lagernuten (16a, 16b) aufweist, welche in der Längsrichtung des Flüssigkeitsspeiseschlauch-Einsetzprofils verlaufen, und wobei das Flüssigkeitsspeiseschlauch-Einsetzprofil einen Boden (18a) aufweist, sowie

eine rolle (13) mit einer Achse (15), die in den Lagernuten derart gelagert oder geführt ist, daß die rolle längs der Lagernuten und längs des Flüssigkeitsspeiseschlauch-Einsetzprofils vom einen Ende zum anderen Ende verschiebbar ist und der Flüssigkeitsspeiseschlauch im Flüssigkeitsspeiseschlauch-Einsetzprofil durch den Umfang der rolle zusammengedrückt oder verquetscht wird,

wobei der Boden des Flüssigkeitsspeiseschlauch-Einsetzprofils erste und zweite Schrägflächen (b, c) aufweist, die gegenüber den Lagernuten geneigt sind und selektiv mit der rolle zusammenzuwirken vermögen, um den Flüssigkeitsspeiseschlauch zur Regelung der Durchflußmenge von Flüssigkeit durch den Flüssigkeitsspeiseschlauch zusammenzudrücken, dadurch gekennzeichnet, daß er ferner eine dritte Schrägfläche (a) aufweist, die ebenfalls gegenüber den Lagernuten geneigt ist und selektiv mit der rolle zusammenzuwirken vermag, um den Flüssigkeitsspeiseschlauch zwecks Regelung der Flüssigkeitsströmungsmenge durch den Flüssigkeitsspeiseschlauch zusammenzudrücken,

die erste Schrägfläche (b) unter einem gegebenen Winkel geneigt und für eine Feineinstellung der Flüssigkeitsströmungsmenge durch den Flüssigkeitsspeiseschlauch geeignet ist, die zweite Schrägfläche (c) mit dem einen Endabschnitt derselben am einen Endabschnitt des ersten Schrägflächenabschnitts endet, der dritte Schrägflächenabschnitt in der gleichen Richtung wie die erste Schrägfläche, aber unter einem größeren Winkel als die erste Schrägfläche geneigt und für eine gröbere Einstellung der Flüssigkeitsströmungsmenge durch den Flüssigkeitsspeiseschlauch geeignet ist, der dritte Schrägflächenabschnitt (a) mit seinem einen Ende an einem anderen Endabschnitt der ersten Schrägfläche (b) endet, so daß der erste Schrägflächenabschnitt für Feineinstellung zwischen den zweiten und dritten Schrägflächenabschnitten für die gröbere Einstellung liegt, und die Länge des ersten Schrägflächenabschnitts größer ist als die Längen der zweiten und dritten Schrägflächenabschnitte.

2. Strömungsregler nach Anspruch 1, bei dem die Neigungswinkel der ersten (b) und zweiten (c) Schrägflächenabschnitte im wesentlichen 1° bzw. 3° betragen.

3. Strömungsregler nach Anspruch 1, bei dem der Neigungswinkel des dritten (c) Schrägflächenabschnitts im wesentlichen 2° beträgt.

4. Strömungsregler nach Anspruch 1, ferner umfassend ein Verdrängungsmittel (20a, 20b, 21a, 21b) zum Verdrängen oder Verdrücken gegenüberliegender Ränder des in das Flüssigkeitsspeiseschlauch-Einsetzprofil eingesetzten Flüssigkeitsspeiseschlauchs, in Längsrichtung des Flüssigkeitsspeiseschlauchs verlaufend.

5. Strömungsregler nach Anspruch 4, bei dem das Verdrängungsmittel durch zwei im wesentlichen kontinuierliche Stege (20a, 20b) gebildet ist, die längs gegenüberliegender Ränder des Bodens (18a) des Flüssigkeitsspeiseschlauch-Einsetzprofils (18) in der Verschieberichtung der rolle (13) und innerhalb eines Verschiebungsbereichs der rolle verlaufen.

**EP 0 337 895 B1**

**6.** Strömungsregler nach Anspruch 4, bei dem das Verdrängungsmittel durch zwei im wesentlichen kontinuierliche Stege (21a, 21b) gebildet ist, die an den gegenüberliegenden rändern des Umfangs der rolle (13) so angeformt sind, daß sie mit dem Flüssigkeitsspeiseschlauch (19) in Berührung stehen.

**7.** Strömungsregler nach Anspruch 4, bei dem das Verdrängungsmittel durch eine Anzahl von im wesentlichen kontinuierlichen Stegen (20a, 20b, 21a, 21b), die an den gegenüberliegenden Rändern des Bodens (18a) des Flüssigkeitsspeiseschlauch-Einsetzprofils (18), in der Verschiebungsrichtung der Rolle (13) verlaufend, und auch an gegenüberliegenden Rändern des Umfangs der Rolle, so daß sie mit dem Flüssigkeitsspeiseschlauch (19) in Berührung stehen, (an)geformt sind.

## Revendications

**1.** Régulateur de débit dans lequel est monté un tube d'apport de liquide souple de telle manière que ledit tube d'apport de liquide souple peut être écrasé du fait de sa souplesse afin de modifier la taille du passage d'écoulement dans ledit tube d'apport de liquide pour réguler le débit de liquide dans ledit tube d'apport de liquide,

ledit régulateur de débit comprenant :

un boîtier (11) comportant un canal d'insertion de tube d'apport de liquide (18) dans lequel est inséré ledit tube d'apport de liquide (19), ledit boîtier présentant des parois latérales opposées (14a, 14b) qui comportent des rainures de support (16a, 16b) qui s'étendent dans la direction longitudinale dudit canal d'insertion de tube d'apport de liquide, ledit canal d'insertion de tube d'apport de liquide ayant un fond (18a), et

un galet (13) comportant un axe (15) soutenu dans lesdites rainures de support de telle manière que ledit galet est déplaçable le long desdites rainures de support et le long dudit canal d'insertion de tube d'apport de liquide d'une extrémité à l'autre extrémité, ledit tube d'apport de liquide dans ledit canal d'insertion de tube d'apport de liquide étant pressé par la périphérie dudit galet,

ledit fond du canal d'insertion de tube d'apport de liquide comportant des première et seconde surfaces inclinées (b, c) qui sont inclinées par rapport auxdites rainures de support et qui coopèrent sélectivement avec ledit galet pour presser ledit tube d'apport de liquide pour réguler le débit de liquide dans ledit tube d'apport de liquide, caractérisé en ce qu'il comprend en outre une troisième surface inclinée (a) qui est inclinée aussi par rapport auxdites rainures de support et qui coopère sélectivement avec ledit galet pour presser ledit tube d'apport de liquide pour réguler le débit de liquide dans ledit tube d'apport de liquide,

ladite première surface inclinée (b) étant inclinée sous un angle donné et étant conçue en vue d'un réglage fin du débit de liquide dans ledit tube d'apport, ladite seconde surface inclinée (c) se terminant à une extrémité au niveau d'une extrémité de ladite première partie de surface inclinée, ladite troisième partie de surface inclinée étant inclinée dans la même direction que ladite première surface inclinée mais sous un plus grand angle que ladite première surface inclinée et étant conçue pour un réglage plus grossier du débit de liquide dans ledit tube d'apport de liquide, ladite troisième partie de surface inclinée (a) se terminant à une extrémité au niveau d'une autre partie extrême de ladite première surface inclinée (b), de telle manière que ladite première partie de surface inclinée pour ledit réglage fin est située entre lesdites seconde et troisième parties de surface inclinée pour ledit réglage plus grossier, et la longueur de ladite première partie de surface inclinée étant supérieure aux longueurs desdites seconde et troisième parties de surface inclinée.

**2.** Régulateur de débit selon la revendication 1, dans lequel les angles d'inclinaison desdites première (b) et seconde (c) parties de surface inclinée sont sensiblement égaux à 1° et 3° respectivement.

**3.** Régulateur de débit selon la revendication 1, dans lequel l'angle d'inclinaison de ladite troisième partie de surface inclinée (c) est sensiblement égal à 2°.

**4.** Régulateur de débit selon la revendication 1, comprenant en outre un dispositif de poussée (20a, 20b, 21a, 21b) pour pousser les bords opposés dudit tube d'apport de liquide inséré dans ledit canal d'insertion, qui s'étend dans la direction longitudinale dudit tube d'apport de liquide.

**5.** Régulateur de débit selon la revendication 4, dans lequel ledit dispositif de poussée est constitué par une paire de nervures (20a, 20b) sensiblement continues qui s'étendent suivant les côtés opposés du fond (18a) dudit canal d'insertion de tube d'apport de liquide (18) dans la direction de déplacement dudit galet (13) et dans une plage de déplacement dudit galet.

6. Régulateur de débit selon la revendication 4, dans lequel ledit dispositif de poussée est constitué par une paire de nervures (21a, 21b) sensiblement continues formées sur les bords opposés de la périphérie dudit galet (13) de manière à être en contact avec ledit tube d'apport de liquide (19).

7. Régulateur de débit selon la revendication 4, dans lequel ledit dispositif de poussée est constitué par une multiplicité de nervures (20a, 20b, 21a, 21b) sensiblement continues formées sur les bords opposés du fond (18a) dudit canal d'insertion de tube d'apport de liquide (18), qui s'étendent dans la direction de déplacement dudit galet (13) et également sur les bords opposés de la périphérie dudit galet de manière à être en contact avec ledit tube d'apport de liquide (19).

# FIG. 1

# FIG.2

# FIG.3

## FIG.4

## FIG.5

# FIG. 6

13
11
21a
21b
19
18a

# FIG. 7

16a  14a  17b
13a
14b
17a  16b
13
12  15
18  11

# FIG. 8

15  13  16a  17a
11
13a  18a  8
a  b  c

FIG. 9